Europäisches Patentamt

European Patent Office

Office européen des brevets

(11) EP 0 534 294 B1

(19)

(12) **EUROPÄISCHE PATENTSCHRIFT**

(45) Veröffentlichungstag und Bekanntmachung des Hinweises auf die Patenterteilung:
**18.12.1996 Patentblatt 1996/51**

(51) Int. Cl.⁶: **C07C 57/055**, C07C 51/25, C07C 51/235

(21) Anmeldenummer: **92115799.6**

(22) Anmeldetag: **16.09.1992**

(54) **Verfahren zur katalytischen Gasphasenoxidation von Acrolein zu Acrylsäure**

Method for the catalytic gas-phase oxidation of acrolein to acrylic acid

Procédé pour l'oxydation catalytique en phase gazeuse d'acroléine en acide acrylique

(84) Benannte Vertragsstaaten:
**BE DE ES FR GB IT**

(30) Priorität: **27.09.1991 DE 4132263**

(43) Veröffentlichungstag der Anmeldung:
**31.03.1993 Patentblatt 1993/13**

(73) Patentinhaber: **BASF Aktiengesellschaft 67063 Ludwigshafen (DE)**

(72) Erfinder:
• **Hammon, Ulrich, Dr.
W-7500 Karlsruhe 1 (DE)**
• **Herzog, Klaus, Dr.
W-6700 Ludwigshafen (DE)**
• **Neumann, Hans-Peter, Dr.
W-6800 Mannheim 31 (DE)**

(56) Entgegenhaltungen:
EP-A- 0 293 859       EP-A- 0 468 290
DE-A- 2 056 614       DE-A- 2 635 031
DE-A- 3 002 829       FR-A- 2 308 609

## Beschreibung

Die vorliegende Erfindung betrifft ein neues Verfahren zur katalytischen Gasphasenoxidation von Acrolein zu Acrylsäure in einem Kontaktrohr-Festbettreaktor bei erhöhter Temperatur an katalytisch aktiven Oxiden mit einem Umsatz des Acroleins bei einfachem Durchgang von $\geq$ 95 %.

Sowohl Acrylsäure für sich, als auch mit niederen Alkoholen verestert, eignet sich als Ausgangsmonomeres zur Herstellung von Polymerisaten verschiedenster Anwendungszwecke (z.B. Klebstoffe).

Die Herstellung von Acrylsäure durch katalytische Gasphasenoxidation von Acrolein verläuft stark exotherm, weshalb es infolge einer Vielfalt von möglichen Parallel- oder Folgereaktionen im Hinblick auf eine möglichst selektive Umsetzung des Acroleins zu Acrylsäure notwendig ist, den Verlauf der Reaktionstemperatur zu steuern.

Aus der DE-A 26 35 031 ist bekannt, bei Acroleinumsätzen bei einfachem Durchgang von über 95 % den Verlauf der Reaktionstemperatur der katalytischen Gasphasenoxidation von Acrolein zu Acrylsäure in einem Kontaktrohr-Festbettreaktor so zu steuern, daß man die Kontaktrohre mit einer 270°C aufweisenden Salzschmelze umgibt.

Aus der DE-A 30 02 829 ist ein zweistufiges Verfahren zur Herstellung von Acrylsäure durch katalytische Gasphasenoxidation von Propylen bekannt, bei dem vor Eintritt in die zweite Oxidationsstufe die Temperatur der Acrolein enthaltenden Reaktionsgase aus der ersten Stufe auf 250°C eingestellt wird und die so temperierten Acrolein enthaltenden Reaktionsgase zur weiteren Oxidation in einen Kontaktrohr-Festbettreaktor geleitet werden, in welchem die Kontaktrohre von einer im Gleichstrom zu den Reaktionsgasen außerhalb der Kontaktrohre fließenden Salzschmelze von 280°C umgeben sind. Aus der DE-A 20 56 614 ist ein zweistufiges Verfahren zur Herstellung von Acrylsäure durch katalytische Gasphasenoxidation von Propylen bekannt, bei dem in der zweiten Oxidationsstufe die Kontaktrohre von einer 250 bis 272°C aufweisenden Salzschmelze umgeben sind, die Acrolein enthaltenden Reaktionsgase aus der ersten Oxidationsstufe der zweiten Oxidationsstufe so zugeführt werden, daß sie bereits die Temperatur des in der zweiten Stufe eingesetzten Salzbades aufweisen und bei dem in der zweiten Oxidationsstufe die Katalysatoraktivität in Strömungsrichtung der Reaktionsgase zunimmt.

Nachteilig an den genannten Verfahren ist, daß die so implizit längs der Kontaktrohre eingestellten Verläufe der Reaktionstemperatur im Hinblick auf eine möglichst selektive Umsetzung des Acroleins zur Acrylsäure nicht voll zu befriedigen vermögen.

Aufgabe der vorliegenden Erfindung war daher, ein Verfahren zur katalytischen Gasphasenoxidation von Acrolein zu Acrylsäure in einem Kontaktrohr-Festbettreaktor bei erhöhter Temperatur an katalytisch aktiven Oxiden mit einem Umsatz des Acroleins bei einfachem Durchgang von $\geq$ 95 % zur Verfügung zu stellen, das einen im Hinblick auf eine erhöhte Selektivität der Acrylsäurebildung verbesserten Verlauf der Reaktionstemperatur aufweist.

Demgemäß wurde ein Verfahren zur katalytischen Gasphasenoxidation von Acrolein zu Acrylsäure in einem Kontaktrohr-Festbettreaktor bei erhöhter Temperatur an katalytisch aktiven Oxiden mit einem Umsatz des Acroleins bei einfachem Durchgang von $\geq$ 95 % gefunden, das dadurch gekennzeichnet ist, daß die Reaktionstemperatur in Strömungsrichtung längs der Kontaktrohre (längs der Reaktionsachse) in einer ersten Reaktionszone ab Eintritt der die Reaktanden enthaltenden Ausgangsreaktionsgase in die Kontaktrohre bis zum Erreichen eines Umsatzes des Acroleins von 20 bis 40 % 260 bis 300°C beträgt und anschließend die Reaktionstemperatur abrupt oder längs der Kontaktrohre bis zum Erreichen eines Acroleinumsatzes von $\geq$ 95 %, d.h. bis zum Austritt der Reaktionsgase aus den Kontaktrohren, sukzessive stufenweise oder kontinuierlich um insgesamt 5 bis 40°C mit der Maßgabe gesenkt wird, daß die Reaktionstemperatur in dieser zweiten Reaktionszone nicht weniger als 240°C beträgt.

Als oxidische Katalysatoren eignen sich unter anderen die Massen die in der EP-A 293 859, der DE-A 22 51 364 und der DE-A 26 26 887 beschrieben sind.

Vorzugsweise werden Massen der allgemeinen Formel I

$$Mo_{12}X_a^1 X_b^2 X_c^3 X_d^4 O_n \qquad (I),$$

in der die Variablen folgende Bedeutung haben:

| | |
|---|---|
| $X^1$: | Vanadin und/oder Wolfram, |
| $X^2$: | Eisen, Mangan und/oder Kupfer, |
| $X^3$: | Niob, Tantal, Titan, Zinn, Antimon und/oder Cer, |
| $X^4$: | Chrom, Cobalt, Zirkonium, Nickel, Silicium und/oder Aluminium, |
| a: | 0,3 bis 6, |
| b: | 0 bis 6, |
| c: | 0 bis 6, |
| d: | 0 bis 6 und |
| n: | eine Zahl, die durch die Wertigkeit und Häufigkeit der vom Sauerstoff verschiedenen Elemente in der allgemeinen Formel I bestimmt wird, |

eingesetzt.

Die genannten oxidischen Katalysatoren sind in an sich bekannter Weise erhältlich. Sie können beispielsweise dadurch hergestellt werden, daß man als Ausgangsverbindungen geeignete Salze der sie konstituierenden elementaren Bestandteile, gegebenenfalls bei erhöhter Temperatur und unter Zusatz von Säuren oder Basen, in wäßrigem Medium durch Lösen und/oder Suspendieren fein verteilt, mischt, das Gemisch trocknet, die erhaltene Masse formt und in der Regel bei Temperaturen von 250 bis 450°C im Luftstrom oder in inerter Atmosphäre, z.B. N₂ oder CO₂, calciniert. Bei der Formung können an sich bekannte Hilfsmittel wie Gleitmittel (z.B. Graphit) oder Formhilfsmittel und Verstärkungsmittel wie Mikrofasern aus Glas, Asbest, Siliciumcarbid oder Kaliumtitanat zugesetzt werden. In dieser Form werden die oxidischen Massen zweckmäßigerweise zur Verwendung als Vollkatalysatoren hergestellt, wobei Hohlzylinder mit einem Außendurchmesser und einer Länge von 4 bis 10 mm und einer Wandstärke von 1 bis 3 mm die bevorzugte Katalysatorgeometrie darstellen. Die katalytisch aktiven Oxide können aber auch in Gestalt von Schalenkatalysatoren, d.h. auf ein vorgeformtes Trägermaterial aufgebracht, angewendet werden, wobei das Aufbringen auf das Trägermaterial z.B. in Form einer wäßrigen Ausgangslösung oder Suspension, verbunden mit anschließender Trocknung und Calcinierung, oder als bereits calcinierte pulverisierte Masse in Kombination mit einem Bindemittel erfolgen kann. Nähere Ausführungen dazu findet man in der DE-A 26 26 887.

Selbstverständlich können die katalytisch aktiven oxidischen Massen auch in Pulverform als Katalysatoren eingesetzt werden.

Der zur Oxidation des Acroleins benötigte Sauerstoff kann z.B. in Form von Luft, aber auch in reiner Form zugesetzt werden. Aufgrund der hohen Reaktionswärme werden die Reaktionspartner vorzugsweise mit Inertgas wie N₂, rückgeführten Reaktionsabgasen und/oder Wasserdampf verdünnt. In der Regel wird bei einem Acrolein:Sauerstoff:Wasserdampf:Inertgas-Verhältnis von 1:(1 bis 3):(0 bis 20):(3 bis 30), vorzugsweise von 1:(1 bis 3):(0,5 bis 10):(7 bis 18) gearbeitet. Normalerweise wird bei dem Verfahren Acrolein eingesetzt, das durch katalytische Gasphasenoxidation von Propen erzeugt wurde. In der Regel werden die Acrolein enthaltenden Reaktionsgase dieser Propenoxidation ohne Zwischenreinigung eingesetzt. Der Reaktionsdruck des erfindungsgemäßen Verfahrens beträgt in der Regel 1 bis 3 bar und die Gesamtraumbelastung beträgt vorzugsweise 1000 bis 2500 Nl/l/h.

Die Realisierung des erfindungsgemäßen Profils der Reaktionstemperatur kann in an sich bekannter Weise erfolgen, z.B. durch abschnittsweise Heizung oder Kühlung des Kontaktrohres mittels elektrischer Temperierbänder oder zirkulierenden fluiden Temperiermedien wie Schmelzen von Salzen wie Kaliumnitrat, Natriumnitrit und/oder Natriumnitrat, oder von niedrig schmelzenden Metallen wie Natrium, Zinn, Quecksilber sowie Legierungen verschiedener Metalle oder Wärmeträgerölen, wobei im Falle eines Einzelrohres infolge des hohen Wärmeübergangs die während der Reaktion im Rohrinneren herrschende Temperatur im wesentlichen gleich der äußeren Heiz- oder Kühltemperatur ist.

Abschnittsweises Heizen oder Kühlen ist aber auch bei Vielrohr-Festbettreaktoren möglich, wie sie zur großtechnischen Realisierung des erfindungsgemäßen Verfahrens vorzugsweise eingesetzt werden, und z.B. in den Schriften DE-A 28 30 765, DE-A 22 01 528, DE-A 16 01 162, DE-A 25 13 405 sowie US-A 3 147 084 beschrieben.

Eine weitere Möglichkeit der Steuerung der Reaktionstemperatur besteht im abschnittsweisen Erhöhen oder Senken der Katalysatoraktivität. Dies kann durch chemische Modifikation der aktiven Katalysatormasse, aber auch durch Verdünnen mit desaktiviertem Katalysator oder Inertmaterial realisiert werden. Gegebenenfalls kann auch abschnittsweises Heizen/Kühlen mit abschnittsweisem Erhöhen/Senken der Katalysatoraktivität kombiniert werden. Vorzugsweise wird bei einem Acroleinumsatz oberhalb von 99 % gearbeitet. Mit besonderem Vorteil wird so gearbeitet, daß im Anschluß an die erste Reaktionszone die Reaktionstemperatur abrupt oder längs der Reaktionsachse bis zum Erreichen des Acroleinendumsatzes sukzessive stufenweise oder kontinuierlich um insgesamt 15 bis 25°C gesenkt wird. Anwendungstechnisch bevorzugt ist eine stufenweise Absenkung, wobei in der Regel 2 bis 4 Stufen angewendet werden.

Da in der großtechnischen Praxis, insbesondere im Falle des mittels Salzbädern temperierten Vielrohr-Festbettreaktors nur ein endlicher Wärmeübergang vom Temperiermedium auf die Reaktionsgase erreicht werden kann, hat es sich als vorteilhaft erwiesen, die Ausgangsreaktionsgase der ersten Reaktionszone vorgeheizt, in der Regel auf Reaktionstemperatur, zuzuführen. Wird längs der ersten Reaktionszone eine Temperatur des Temperiermediums oberhalb 260 aber ≦ 280°C gewählt, werden die Reaktionsgase der ersten Reaktionszone vorzugsweise auf eine Temperatur vorgeheizt zugeführt, die 15 bis 20°C oberhalb der Temperatur liegt, die das Temperiermedium am Beginn der ersten Reaktionszone aufweist. Liegt die Temperatur des Temperiermediums längs der ersten Reaktionszone im Bereich > 280 ≦ 300°C, werden die Reaktionsgase der ersten Reaktionszone vorzugsweise auf eine Temperatur vorgeheizt zugeführt, die lediglich bis zu 10°C oberhalb der Temperatur liegt, die das Temperiermedium am Beginn der ersten Reaktionszone aufweist. Ist die Temperatur des Temperiermediums längs der ersten Reaktionszone konstant, stellt sich so in Strömungsrichtung längs der ersten Reaktionszone eine kontinuierlich fallende Reaktionstemperatur ein. Dies hat sich als besonders vorteilhaft erwiesen, insbesondere dann, wenn der kontinuierliche Abfall der Reaktionstemperatur längs der zweiten Reaktionszone in Strömungsrichtung fortgesetzt wird. Selbstverständlich kann der kontinuierliche Abfall der Reaktionstemperatur längs der Reaktionsachse durch sukzessive stufenweise Temperaturabnahme angenähert werden. Vorzugsweise beträgt der Abfall der Reaktionstemperatur längs der ersten Reaktionszone in Strömungsrichtung 5 bis 20°C.

Typische Kontaktrohre bestehen aus korrosions- und hitzebeständigem Stahl (z.B. V2A) einer Wanddicke von etwa 2 mm und einem Innendurchmesser von 25 mm. Die Anzahl solcher Kontaktrohre in einem Vielrohr-Festbettreaktor beläuft sich in der Regel auf 10000 bis 40000. Umsatz U und Selektivität S sind in dieser Schrift wie folgt definiert:

$$U = \frac{\text{Molzahl umgesetztes Acrolein}}{\text{Molzahl eingesetztes Acrolein}} \times 100$$

$$S = \frac{\text{Molzahl gebildeter Acrysäure}}{\text{Molzahl umgesetztes Acrolein}} \times 100 \ .$$

Beispiele B1 bis B4 und Vergleichsbeispiele V1 bis V2

Ein mittels elektrischer Heizbänder abschnittsweise temperiertes Stahlrohr (V2A, 2 mm Wanddicke, 25 mm Innendurchmesser) wurde mit einem Katalysator entsprechend Beispiel 1 der DE-A 26 26 887 bis zu einer Füllhöhe von 3 m gefüllt und mit 2400 Nl/l/h einer Gasmischung der Zusammensetzung

4,5 Vol.%      Acrolein,
6,5 Vol.%      Sauerstoff,
10,0 Vol.%      Wasserdampf und
79,0 Vol.%      Stickstoff,

die je nach Beispiel auf verschiedene Temperaturen $T^1$ vorgeheizt war, beschickt. Anschließend wurde längs der ersten Reaktionszone bis zu einem Acroleinumsatz von 40 % die Temperatur der elektrischen Heizbänder auf $T^2$ und danach bis zum Verlassen der Reaktionsgase auf $T^3$ eingestellt. Der Endumsatz $U^{End}$ wurde durch die Länge der zweiten Reaktionszone festgelegt. Die erhaltenen Ergebnisse (Selektivität $S^{End}$ der Acrylsäurebildung) weist die Tabelle aus.

Tabelle

|    | $T^1$ | $T^2$ | $T^3$ | $U^{End}$ | $S^{End}$ |
|----|-------|-------|-------|-----------|-----------|
| B1 | 280   | 280   | 266   | 99,2      | 96,8      |
| B2 | 285   | 285   | 264   | 99,4      | 97,5      |
| B3 | 290   | 290   | 258   | 99,1      | 96,1      |
| B4 | 290   | 285   | 263   | 99,1      | 97,3      |
| V1 | 270   | 270   | 270   | 99,2      | 95,4      |
| V2 | 250   | 250   | 298   | 99,3      | 94,8      |

**Patentansprüche**

1. Verfahren zur katalytischen Gasphasenoxidation von Acrolein zu Acrylsäure in einem Kontaktrohr-Festbettreaktor bei erhöhter Temperatur an katalytisch aktiven Oxiden mit einem Umsatz des Acroleins bei einfachem Durchgang von ≧ 95 %, dadurch gekennzeichnet, daß die Reaktionstemperatur in Strömungsrichtung längs der Kontaktrohre in einer ersten Reaktionszone ab Eintritt der die Reaktanden enthaltenden Ausgangsreaktionsgase in die Kontaktrohre bis zum Erreichen eines Umsatzes des Acroleins von 20 bis 40 % 260 bis 300°C beträgt und anschließend die Reaktionstemperatur abrupt oder längs der Kontaktrohre bis zum Erreichen eines Acroleinumsatzes von ≧ 95 % sukzessive stufenweise oder kontinuierlich um insgesamt 5 bis 40°C mit der Maßgabe gesenkt wird, daß die Reaktionstemperatur in dieser zweiten Reaktionszone nicht weniger als 240°C beträgt.

2. Verfahren nach Anspruch 1, dadurch gekennzeichnet, daß die Reaktionstemperatur der Reaktionsgase in Strömungsrichtung längs der Kontaktrohre bis zum Verlassen der Kontaktrohre sukzessive abnimmt.

3. Verfahren nach Anspruch 1 oder 2, dadurch gekennzeichnet, daß als oxidische Katalysatoren Massen der allgemeinen Formel I

$$Mo_{12}X^1_a X^2_b X^3_c X^4_d O_n \qquad (I),$$

in der die Variablen folgende Bedeutung haben:

$X^1$:      Vanadin und/oder Wolfram,
$X^2$:      Eisen, Mangan und/oder Kupfer,
$X^3$:      Niob, Tantal, Titan, Zinn, Antimon und/oder Cer,
$X^4$:      Chrom, Cobalt, Zirkonium, Nickel, Silicium und/oder Aluminium,
a:      0,3 bis 6,
b:      0 bis 6,
c:      0 bis 6,
d:      0 bis 6 und
n:      eine Zahl, die durch die Wertigkeit und Häufigkeit der von Sauerstoff verschiedenen Elemente in der allgemeinen Formel I bestimmt wird,

eingesetzt werden.

**Claims**

1. A process for the catalytic gas-phase oxidation of acrolein to acrylic acid in a fixed-bed reactor with contacting tubes, at elevated temperature on catalytically active oxides with a conversion of acrolein for a single pass of ≧ 95%, wherein the reaction temperature in the flow direction along the contacting tubes in a first reaction zone before the starting reaction gases containing the reactants enter the contacting tubes is from 260 to 300°C until an acrolein conversion of from 20 to 40% is reached, and the reaction temperature is subsequently reduced by a total of from 5 to 40°C, abruptly or successively in steps or continuously along the contacting tubes until an acrolein conversion of ≧ 95% has been reached, with the proviso that the reaction temperature in this secondary reaction zone is not lower than 240°C.

2. A process as claimed in claim 1, wherein the reaction temperature of the reaction gases drops successively in the flow direction along the contacting tubes until they leave the contacting tubes.

3. A process as claimed in claim 1 or 2, wherein the oxidic catalysts are materials of the formula I

$$Mo_{12}X^1_a X^2_b X^3_c X^4_d O_n \qquad (I),$$

where

$X^1$ is vanadium and/or tungsten,
$X^2$ is iron, manganese and/or copper,
$X^3$ is niobium, tantalum, titanium, tin, antimony and/or cerium,
$X^4$ is chromium, cobalt, zirconium, nickel, silicon and/or aluminum,
a is 0.3 to 6,
b is 0 to 6,
c is 0 to 6,
d is 0 to 6 and
n is a number determined by the valency and frequency of the elements other than oxygen in the formula I.

**Revendications**

1. Procédé d'oxydation en phase gazeuse et catalytique de l'acroléine en acide acrylique dans un réacteur à tube de contact-lit fixe, à température élevée, sur des oxydes catalytiquement actifs avec une conversion de l'acroléine à une seule passe égale ou supérieure à 95%, caractérisé en ce que la température réactionnelle en direction de l'écoulement le long des tubes de contact dans une première zone réactionnelle depuis l'entrée des gaz réactionnels de départ contenant les réactifs dans les tubes de contact jusqu'à l'obtention d'une conversion de l'acroléine de 20 à 40%, varie de 260 à 300°C et en ce que, ensuite, on abaisse la température réactionnelle de manière abrupte ou le long des tubes de contact jusqu'à l'obtention d'une conversion de l'acroléine égale ou supérieure à 95%, par échelons successifs ou de manière continue, d'au total 5 à 40°C, avec la condition que la température réactionnelle dans cette seconde zone de réaction ne soit pas inférieure à 240°C.

2. Procédé suivant la revendication 1, caractérisé en ce que la température réactionnelle des gaz réactionnels diminue par échelons dans la direction de l'écoulement le long des tubes de contact jusqu'à ce qu'ils quittent les tubes de contact.

3. Procédé suivant la revendication 1 ou 2, caractérisé en ce que l'on utilise des masses de catalyseurs oxydiques de la formule générale I

$$Mo_{12}X^1_a X^2_b X^3_c X^4_d O_n \qquad (I),$$

dans laquelle les variables ont les significations suivantes :

$X^1$: vanadium et/ou tungstène,
$X^2$: fer, manganèse et/ou cuivre,
$X^3$: niobium, tantale, titane, étain, antimoine et/ou cérium,
$X^4$: chrome, cobalt, zirconium, nickel, silicium et/ou aluminium,
a: 0,3 à 6,
b: O à 6,
c: 0 o 6,
d: O à 6 et
n: un nombre qui est déterminé par la valence et la fréquence des éléments qui diffèrent de l'oxygène dans la formule générale I.